# EUROPEAN PATENT APPLICATION

(11) **EP 1 815 866 A1**
(43) Date of publication of application: **08.08.2007**
(21) Application number: 05800236.1
(22) Date of filing: 07.11.2005
(51) Int. Cl.: A61K 45/00, A61K 31/496, A61K 38/00, A61K 48/00, C07D 471/04, A61P 9/10

(54) **REMEDY OR PREVENTIVE FOR ISCHEMIC CARDIAC DISEASE OR ISCHEMIC CARDIAC MYOPATHY**

(30) Priority: 10.11.2004 JP 2004326234
(71) Applicant: Neugen Pharma, Inc, Tokyo 1130021 (JP)
(72) Inventor: YOTSUYA, Shuichi, ISHIHARA SANGYO KAISHA, LTD., Kusatsu-shi, Shiga 5250025 (JP); KIMURA, Hirohiko, ISHIHARA SANGYO KAISHA, LTD., Kusatsu-shi, Shiga 5250025 (JP); IKEDA, Joh-E, Tokyo 1530051 (JP)
(74) Representative: Grünecker, Kinkeldey, Stockmair & Schwanhäusser Anwaltssozietät
(86) International application number: PCT/JP2005/020381
(87) International publication number: WO 2006/051757

(57) **Abstract**

[Problems to be Solved] The present invention provides therapeutic or preventive agents for ischemic heart disease or ischemic cardiomyopathy, where the agents suppress the progress of myocardial necrosis due to functional damage of cardiac tissues that have undergone reperfusion injury, or where the agents have protective activity towards cardiac tissues.

[Means to Solve the Problems] Therapeutic or preventive agents for ischemic heart disease or ischemic cardiomyopathy, which comprise NAIP gene upregulator compounds as active ingredients.

## Description

### Technical Field

The present invention relates to therapeutic or preventive agents for ischemic heart disease or ischemic cardiomyopathy, where the agents comprise upregulator compounds for NAIP genes, such as specific 3-substituted (aza)indole compounds, as active ingredients.

### Background Art

Patients with cardiovascular disease who suffer from ischemic heart disease, such as myocardial infarctions, have rapidly increased in recent years. Therapeutic methods that actively reopen obstructed coronary arteries, such as coronary angioplasty (PTCA) or intracoronary thrombolytic therapy, have come into widespread use as therapeutic methods for such myocardial infarctions. However, although these therapeutic methods are essential for myocardial recovery from ischemia, it is now known that new damage can develop as the blood flow resumes; this damage includes ventricular arrhythmia such as ventricular fibrillation, hemorrhagic infarction, and no-reflow phenomenon, and can lead to worse conditions. This has become known as reperfusion injury. The function of cell membranes in cardiac tissues damaged in this way is thought to begin to break down, and myocardial necrosis is thought to progress.

Therefore, an important issue in the treatment of ischemic heart disease or ischemic cardiomyopathy is to deal with the development of reperfusion injuries.

Patent Document 1 reveals that substituted pyrrolo[2,3-b]-pyridine derivatives show selective affinity to dopamine D4 receptor subtype in the brain, and are therefore useful for treating and preventing psychiatric disorders.

Patent Document 2 discloses pyrrolo-pyridine derivatives that are said to show suppression of menadione-induced cell death, suppression of deformation/deciduation of nerve cells in ischemia-reperfused gerbil hippocampal tissue, and survival benefits in amyotrophic lateral sclerosis (ALS) model mice; these derivatives are thus said to be useful for treating and preventing neurodegenerative diseases.

Patent Document 3 discloses piperazine derivatives that have suppressive effects against the autonomic nervous system, cardiovascular system, and skeletal muscle system; these derivatives are said to be useful as tranquilizers, sedatives, adrenolytic agents, hypothermic agents, anticonvulsants, hypotensive agents, and cardiovascular agents. Patent Document 4 discloses piperazine derivatives that have suppressive effects against the autonomic nervous system, cardiovascular system, and skeletal muscle system, and are said to be useful as tranquilizers, sedatives, skeletal muscle relaxants, adrenolytic agents, hypothermic agents, anticonvulsants, hypotensive agents, and cardiovascular agents.

Patent Document 5 discloses pyrrolo-pyridine derivatives; since these compounds are ligands of dopamine receptor subtypes, they are said to be useful for treating and preventing dopamine-related diseases, particularly schizophrenia. Since rat dopamine receptor mRNA is localized in the heart and great vessels, Patent Document 5 suggests that dopamine receptor ligands may control cardiovascular functions by influencing the contractility of the heart and smooth muscles, or by regulating the secretion of vasoactive substances; thus, these compounds may be useful for preventing and treating hypertension and congestive heart failure.

Non-Patent Document 1 describes the neuronal apoptosis inhibitory protein (NAIP) gene; Non-Patent Document 2 describes that the death of various cultured cells subjected to apoptosis-inducing stimuli is inhibited when these cells have been introduced with NAIP gene; and Patent Document 6 describes a complete cDNA of NAIP gene and a protein encoded by the cDNA.

As described above, these prior arts neither discloses nor suggests that the compounds used in the present application are useful for treating or preventing ischemic heart disease or ischemic cardiomyopathy.
[Patent Document 1] International Publication WO94/20497
[Patent Document 2] International Publication WO2004/028540
[Patent Document 3] U.S. Patent Publication US3362956
[Patent Document 4] U.S. Patent Publication US3511841
[Patent Document 5] International Publication WO94/20459
[Patent Document 6] Japanese Patent Application Kokai Publication No. (JP-A) H11-116599 (unexamined, published Japanese patent application)
[Non-Patent Document 1] Roy et al., Cell vol. 80: pp.167-178, 1995
[Non-Patent Document 2] Liston et al., Nature vol. 379: pp.349-353, 1996

### Disclosure of the Invention

### [Problems to be Solved by the Invention]

An objective of the present invention is to provide therapeutic or preventive agents for ischemic heart disease or ischemic cardiomyopathy, where the agents suppress the progress of myocardial necrosis due to the functional damage of cardiac tissues that have undergone reperfusion injury, or where the agents have a protective activity towards cardiac tissues.

### [Means to Solve the Problems]

The present inventors conducted research to solve the above-mentioned problems, and as a result they discovered that compositions comprising upregulator compounds for NAIP genes have protective activity towards the affected tissues in cardiac ischemia-reperfusion animal models, and they thus completed the present invention.
(1) The present invention relates to therapeutic or preventive agents for ischemic heart disease or ischemic cardiomyopathy, where the agents comprise upregulator compounds for NAIP genes as active ingredients.
(2) The above-described upregulator compounds for NAIP genes are, more specifically, compounds represented by formula (I) shown below, or salts thereof: 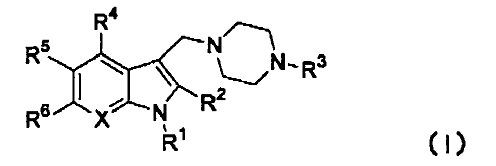 (wherein,
   X represents CH or a nitrogen;
   R¹ and R² independently represent a hydrogen or a C₁₋₆ alkyl;
   R³ represents an optionally substituted phenyl, optionally substituted pyridyl, optionally substituted quinolyl, optionally substituted isoquinolyl, optionally substituted pyridazinyl, optionally substituted pyrimidinyl, optionally substituted pyrazinyl, optionally substituted pyranyl, optionally substituted furyl, optionally substituted benzofuryl, optionally substituted dibenzofuryl, optionally substituted thienyl, optionally substituted benzothienyl, optionally substituted indolyl, optionally substituted indazolyl, optionally substituted imidazolyl, optionally substituted benzimidazolyl, optionally substituted oxadiazolyl, optionally substituted thiadiazolyl, or optionally substituted C₃₋₇ cycloalkyl;
   R⁴, R⁵, and R⁶ each independently represent a hydrogen, C₁₋₆ alkyl, halogen, cyano, trifluoromethyl, nitro, -OR⁷, -SR⁷, -SO₂R⁷, -SOR⁷, -SO₂NR⁷R⁸, -NR⁷R⁸, -NR⁷SO₂R⁸, -COR⁷, -CO₂R⁷, or -CONR⁷R⁸; and
   R⁷ and R⁸ each independently represent a hydrogen or a C₁₋₆ alkyl).
(3) The agents of (2), wherein in the aforementioned formula (I), R³ is a phenyl optionally substituted with Y, a pyridyl optionally substituted with Y, a quinolyl optionally substituted with Y, an isoquinolyl optionally substituted with Y, a pyridazinyl optionally substituted with Y, a pyrimidinyl optionally substituted with Y, a pyrazinyl optionally substituted with Y, a pyranyl optionally substituted with Y, a furyl optionally substituted with Y, a benzofuryl optionally substituted with Y, a dibenzofuryl optionally substituted with Y, a thienyl optionally substituted with Y, a benzothienyl optionally substituted with Y, an indolyl optionally substituted with Y, an indazolyl optionally substituted with Y, an imidazolyl optionally substituted with Y, a benzimidazolyl optionally substituted with Y, an oxadiazolyl optionally substituted with Y, a thiadiazolyl optionally substituted with Y, or a C₃₋₇ cycloalkyl optionally substituted with Y;
   wherein Y is at least one substituent selected from the group consisting of a C₁₋₆ alkyl, phenyl, phenoxy, benzyl, benzyloxy, halogen, trifluoromethyl, nitro, cyano, -OR⁹, -SR⁹, -SO₂R⁹, -SOR⁹, -SO₂NR⁹R¹⁰, -NR⁹R¹⁰, -NR⁹SO₂R¹⁰, -COR⁹, -CO₂R⁹, and -CONR⁹R¹⁰; and
   R⁹ and R¹⁰ each independently represent a hydrogen or C₁₋₆ alkyl.
(4) The agents of (2) or (3), wherein in the aforementioned formula (I), R³ is a phenyl optionally substituted with Y, a pyridyl optionally substituted with Y, an isoquinolyl optionally substituted with Y, a thienyl optionally substituted with Y, or a C₃₋₇ cycloalkyl optionally substituted with Y
(5) The agents of any of (2) to (4), wherein in the aforementioned formula (1), R³ is a phenyl optionally substituted with Y
(6) The agents of any of (2) to (5), wherein in the aforementioned formula (I), X represents a nitrogen.
(7) The agents of any of (1) to (6), which comprise as an active ingredient 3-[4-(4-chlorophenyl)piperazin-1-yl]methyl-1H-pyrrolo[2,3-b]pyridine or salts thereof.
(8) Methods for treating or preventing ischemic heart disease or ischemic cardiomyopathy, which comprise administering patients with effective doses of upregulator compounds for NAIP genes.
(9) The methods of (8), wherein the upregulator compounds for NAIP genes are compounds represented by the aforementioned formula (I), or salts thereof.
(10) The methods of (9) which comprise administering compounds represented by the aforementioned formula (1), or salts thereof, wherein in formula (I), R³ is a phenyl optionally substituted with Y, a pyridyl optionally substituted with Y, a quinolyl optionally substituted with Y, an isoquinolyl optionally substituted with Y, a pyridazinyl optionally substituted with Y, a pyrimidinyl optionally substituted with Y, a pyrazinyl optionally substituted with Y, a pyranyl optionally substituted with Y, a furyl optionally substituted with Y, a benzofuryl optionally substituted with Y, a dibenzofuryl optionally substituted with Y, a thienyl optionally substituted with Y, a benzothienyl optionally substituted with Y, an indolyl optionally substituted with Y, an indazolyl optionally substituted with Y, an imidazolyl optionally substituted with Y, a benzimidazolyl optionally substituted with Y, an oxadiazolyl optionally substituted with Y, a thiadiazolyl optionally substituted with Y, or a C₃₋₇ cycloalkyl optionally substituted with Y;
   wherein Y is at least one substituent selected from the group consisting of a C₁₋₆ alkyl, phenyl, phenoxy, benzyl, benzyloxy, halogen, trifluoromethyl, nitro, cyano, -OR⁹, -SR⁹, -SO₂R⁹, -SOR⁹, -SO₂NR⁹R¹⁰, -NR⁹R¹⁰, -NR⁹SO₂R¹⁰, -COR⁹, -CO₂R⁹, and -CONR⁹R¹⁰; and
   R⁹ and R¹⁰ each independently represent a hydrogen or a C₁₋₆ alkyl.
(11) The methods of (9) or (10), which comprise administering compounds represented by the aforementioned formula (I) or salts thereof, wherein in formula (I), R³ is a phenyl optionally substituted with Y, a pyridyl optionally substituted with Y, an isoquinolyl optionally substituted with Y, a thienyl optionally substituted with Y, or a C₃₋₇ cycloalkyl optionally substituted with Y .
(12) The methods of any of (9) to (11), which comprise administering compounds represented by the aforementioned formula (I) or salts thereof, wherein in formula (I), R³ is a phenyl optionally substituted with Y
(13) The methods of any of (9) to (12), which comprise administering compounds represented by the aforementioned formula (I) or salts thereof, wherein in formula (I), X is a nitrogen.
(14) The methods of any of (8) to (13), which comprise administering 3-[4-(4-chlorophenyl)piperazin-1-yl]methyl-1H-pyrrolo[2,3-b]pyridine or salts thereof.
(15) Use of upregulator compounds for NAIP genes for producing therapeutic or preventive agents for ischemic heart disease or ischemic cardiomyopathy.
(16) The use of (15), wherein the upregulator compounds for NAIP genes are compounds represented by the aforementioned formula (I), or salts thereof.
(17) The use of (16), wherein in the aforementioned formula (I), R³ is a phenyl optionally substituted with Y, a pyridyl optionally substituted with Y, a quinolyl optionally substituted with Y, an isoquinolyl optionally substituted with Y, a pyridazinyl optionally substituted with Y, a pyrimidinyl optionally substituted with Y, a pyrazinyl optionally substituted with Y, a pyranyl optionally substituted with Y, a furyl optionally substituted with Y, a benzofuryl optionally substituted with Y, a dibenzofuryl optionally substituted with Y, a thienyl optionally substituted with Y, a benzothienyl optionally substituted with Y, an indolyl optionally substituted with Y, an indazolyl optionally substituted with Y, an imidazolyl optionally substituted with Y, a benzimidazolyl optionally substituted with Y, an oxadiazolyl optionally substituted with Y, a thiadiazolyl optionally substituted with Y, or a C₃₋₇ cycloalkyl optionally substituted with Y;
   wherein Y is at least one substituent selected from the group consisting of a C₁₋₆ alkyl, phenyl, phenoxy, benzyl, benzyloxy, halogen, trifluoromethyl, nitro, cyano, -OR⁹, -SR⁹, -SO₂R⁹, -SOR⁹, -SO₂NR⁹R¹⁰, -NR⁹R¹⁰, -NR⁹SO₂R¹⁰, -COR⁹, -CO₂R⁹, and -CONR⁹R¹⁰; and
   R⁹ and R¹⁰ each independently represent a hydrogen or a C₁₋₆ alkyl.
(18) The use of (16) or (17), wherein in the aforementioned formula (I), R³ is a phenyl optionally substituted with Y, a pyridyl optionally substituted with Y, an isoquinolyl optionally substituted with Y, a thienyl optionally substituted with Y, or a C₃₋₇ cycloalkyl optionally substituted with Y.
(19) The use of any of (16) to (18), wherein in the aforementioned formula (I). R³ is a phenyl optionally substituted with Y
(20) The use of any of (16) to (19), wherein in the aforementioned formula (I), X is a nitrogen.
(21) The use of any of (15) to (20), wherein
   3-[4-(4-chlorophenyl)piperazin-1-yl]methyl-1H-pyrrolo[2,3-b]pyridine or salts thereof are used.
(22) Therapeutic or preventive agents for ischemic heart disease or ischemic cardiomyopathy that comprise as active ingredients any of the following substances:
   (a) NAIP proteins;
   (b) NAIP protein mutants functionally equivalent to NAIP protein and comprising an amino acid sequence with one or more amino acid deletions, substitutions, or additions in the amino acid sequence of NAIP protein; or
   (c) DNAs encoding the proteins of (a) or (b) described above, or vectors that express said proteins.
(23) Therapeutic or preventive methods for ischemic heart disease or ischemic cardiomyopathy which comprise administering patients with effective doses of any of the following substances:
   (a) NAIP proteins;
   (b) NAIP protein mutants functionally equivalent to NAIP protein and comprising an amino acid sequence with one or more amino acid deletions, substitutions, or additions in the amino acid sequence of NAIP protein; or
   (c) DNAs encoding the proteins of (a) or (b) described above, or vectors that express said proteins.
(24) Use of any of the substances (a) to (c) described below for producing therapeutic or preventive agents for ischemic heart disease or ischemic cardiomyopathy:
   (a) NAIP proteins;
   (b) NAIP protein mutants functionally equivalent to NAIP protein and comprising an amino acid sequence with one or more amino acid deletions, substitutions, or additions in the amino acid sequence of NAIP protein; or
   (c) DNAs encoding the proteins of (a) or (b) described above, or vectors that express said proteins.

### [Effects of the Invention]

The therapeutic or preventive agents of the present invention can suppress reperfusion injuries caused by ischemia-reperfusion when administered to ischemic heart disease or ischemic cardiomyopathy patients, or to patients who have received blood reflow treatments such as coronary angioplasties or coronary artery thrombolysis. In particular, the agents can suppress cardiac tissue edema, hyperemia/congestion, bleeding, and neutrophil infiltration. They can also reduce the degree or frequency with which myocardial degenerative changes occur, such as the edematous degeneration, wavy fiber pattern, and contraction band necrosis that take place in such cardiac tissues. In addition, the agents can reduce the degree or frequency with which coagulation necrosis and necrobiosis occur. Furthermore, they can reduce the degree or frequency with which apoptosis takes place in such cardiac tissues.

### Brief Description of the Drawings

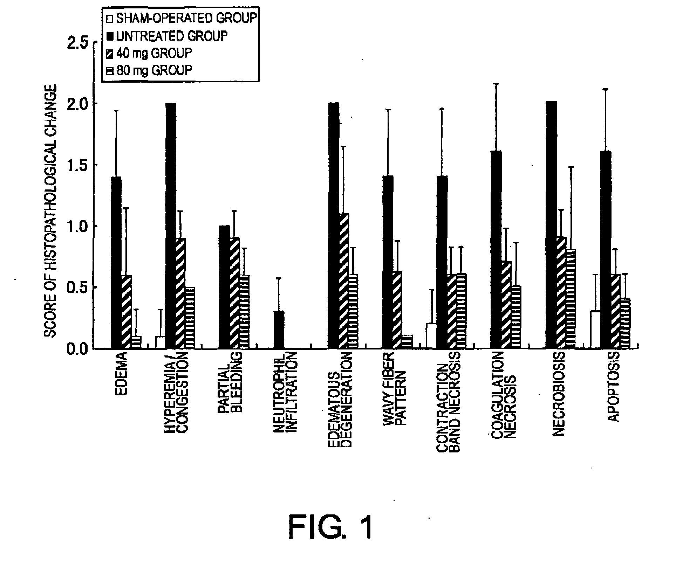

Fig. 1 shows the scores for histopathological changes in each of the lesions observed at the left ventricular wall for each group in the Test Example.

### Best Mode for Carrying Out the Invention

The present inventors discovered that upregulation (activation) of expression of Neuronal Apoptosis Inhibitory Protein (NAIP) genes can treat or prevent ischemic heart disease or ischemic cardiomyopathy. More specifically, upregulator compounds (expression enhancers) for NAIP genes are useful as therapeutic or preventive agents for ischemic heart disease or ischemic cardiomyopathy.

The present invention provides therapeutic or preventive agents for ischemic heart disease or ischemic cardiomyopathy that comprise upregulator compounds for NAIP genes as active ingredients.

The "upregulator compounds" of the present invention include compounds that activate NAIP gene transcription and/or compounds that activate translation of the transcription products of NAIP genes.

The upregulator compounds of the present invention include, for example, substances with the function of acting on the transcriptional regulatory domain (promoters and such) of NAIP genes to increase the transcriptional activity of these genes. For example, transcription factors (transcriptional activators) of NAIP genes are preferred upregulator compounds of the present invention.

In the present invention, NAIP gene expression activity can be suitably measured using methods well known to those skilled in the art, such as Northern and Western blotting.

The present inventors discovered that compounds with the activity of upregulating NAIP gene expression effectively suppress the apoptosis caused by oxidative stress/radical-inducing agents, and have therapeutic effects against ischemic heart disease or ischemic cardiomyopathy. More specifically, by increasing the amount ofNAIP protein present in cells, ischemic heart disease or ischemic cardiomyopathy can be effectively treated.

Therefore, NAIP proteins themselves, or substances that activate the function of these proteins (function enhancers) are useful as therapeutic or preventive agents for ischemic heart disease or ischemic cardiomyopathy.

Examples of the NAIP protein function enhancers of the present invention include:
(a) NAIP proteins;
(b) NAIP protein mutants functionally equivalent to NAIP proteins and comprising an amino acid sequence with one or more amino acid deletions, substitutions, or additions in the amino acid sequence of NAIP protein; and
(c) DNAs encoding the proteins of the above (a) or (b), or vectors expressing such proteins.

The NAIP proteins in the present invention are ordinarily proteins derived from the organisms in which ischemic heart disease or ischemic cardiomyopathy is to be treated or prevented (endogenous proteins). The NAIP proteins of the present invention are preferably human NAIP proteins, but the organisms from which these proteins are derived are not particularly limited, and examples include the NAIP proteins of mammals such as mice. For example, the amino acid sequence of a NAIP protein and a DNA sequence encoding this protein are described in WO96/12016.

The "NAIP proteins" of the present invention can be prepared as naturally occurring proteins as well as recombinant proteins using gene-recombination technology. Naturally occurring proteins can be prepared, for example, by methods in which extracts of cells (tissues) considered to be expressing a NAIP protein are subjected to affinity chromatography using antibodies against NAIP protein. On the other hand, recombinant proteins can be prepared by culturing cells transformed with DNAs encoding NAIP proteins.

In the present invention, "functionally equivalent proteins" refer to proteins with activities similar to the activities revealed for NAIP protein. Examples of the above "activities" include the activity of suppressing the apoptosis caused by oxidative stress-inducing compounds.

Furthermore, the NAIP proteins of the present invention include proteins that are functionally equivalent to a NAIP protein and that have sequence homology of, for example, 70% or greater, preferably 90% or greater, more preferably 95% or greater, and even more preferably 98% or greater to the amino acid sequence of a NAIP protein. Furthermore, amino acid modification in the present invention ordinarily involves 50 amino acids or less, preferably 30 amino acids or less, more preferably ten amino acids or less, or even more preferably three amino acids or less.

Examples of the above functionally equivalent proteins include human NAIP protein mutants, and human NAIP protein homologs found in organisms other than humans.

The DNAs encoding the NAIP proteins of the present invention may be chromosomal DNAs or cDNAs. Chromosomal DNAs encoding NAIP proteins can be obtained, for example, by preparing chromosomal DNA libraries from cells and such, and screening these libraries using probes that hybridize to DNAs that encode NAIP proteins. cDNAs encoding NAIP proteins can be obtained by extracting RNA samples from cells (tissues) considered to be expressing a NAIP protein, and performing gene amplification techniques such as RT-PCR using primers that hybridize to DNAs encoding NAIP proteins.

The NAIP proteins of the present invention or DNAs encoding these proteins may be mutants whose nucleotide sequence or amino acid sequence has been modified, so long as they are functionally equivalent to a NAIP protein. Such mutants may be naturally or artificially derived. Methods for artificially preparing mutants are well known to those skilled in the art. Known examples include the Kunkel method (Kunkel, T. A. et al., Methods Enzymol. 154, 367-382 (1987)), double primer method (Zoller, M. J. and Smith, M., Methods Enzymol. 154, 329-350 (1987)), cassette mutation method (Wells, et al., Gene 34, 315-23 (1985)), and megaprimer method (Sarkar, G and Sommer, S. S., Biotechniques 8, 404-407 (1990)).

The DNAs encoding the NAIP proteins of the present invention are preferably functionally linked to promoters for efficient expression. Original NAIP gene promoters may be used as such promoters, but in addition, various known promoters such as CMV promoters and such may be used. Furthermore, those skilled in the art can easily produce vectors that express the above-mentioned proteins using a variety of known expression vectors.

The above-mentioned vectors can be used in gene therapy. Gene therapy refers to treatment or prevention through administration to patients of vectors comprising DNAs encoding functional proteins. Examples of vectors that may be used for gene therapy include adenovirus vectors or retrovirus vectors, but are not limited thereto. General gene manipulations such as insertion of a DNA encoding the above-mentioned protein into a vector can be performed according to conventional methods.

Preferred embodiments of the above-mentioned "upregulator compounds" of the present invention include compounds represented by the aforementioned formula (I).

In formula (I) mentioned above, the C₁₋₆ alkyl includes straight, and branched chain alkyls, and specific examples include a methyl, ethyl, propyl, isopropyl, butyl, tert-butyl, pentyl, and hexyl.

Specific examples of C₃₋₇ cycloalkyl include cyclopropyl, cyclobutyl, cyclopentyl, cyclohexyl, and cycloheptyl.

Phenyl, pyridyl, quinolyl, isoquinolyl, pyridazinyl, pyrimidinyl, pyrazinyl, pyranyl, furyl, benzofuryl, dibenzofuryl, thienyl, benzothienyl, indolyl, indazolyl, imidazolyl, benzimidazolyl, oxadiazolyl, thiadiazolyl, or C₃₋₇ cycloalkyl included within R³ can be optionally substituted with Y

The aforementioned Y preferably includes a C₁₋₆ alkyl phenyl, phenoxy, benzyl, benzyloxy, halogen, trifluoromethyl, nitro, cyano, -OR⁹, -SR⁹, -SO₂R⁹, -SOR⁹, -SO₂NR⁹R¹⁰,
- NR⁹R¹⁰, -NR⁹SO₂R¹⁰, -COR⁹, -CO₂R⁹, and -CONR⁹R¹⁰.

The aforementioned Y is more preferably a C₁₋₆ alkyl, phenyl, phenoxy, benzyl, benzyloxy, halogen, trifluoromethyl, cyano, -OR⁹, -SR⁹, -SO₂R⁹, -NR⁹R¹⁰, or -NR⁹SO₂R¹⁰.

There may be one or two or more substituent Ys, and, there is preferably one or two. When there are two or more, they may be the same or different. The substitution may be at any site.

The aforementioned R⁹ and R¹⁰ each individually represent a hydrogen or a C₁₋₆ alkyl. Specific examples of halogens included within R⁴, R⁵, R⁶, or Y include fluorines, chlorines, bromines, or iodines.

R⁴, R⁵, or R⁶ is as described above, but is more preferably a hydrogen, C₁₋₆ alkyl, halogen, cyano, -OR⁷, -SO₂R⁷, -SO₂NR⁷R⁸, -NR⁷R⁸, or -NR⁷SO₂R⁸.

R¹ or R² is as described above, but is more preferably a hydrogen or a methyl group, and even more preferably a hydrogen.

Examples of compounds represented by the aforementioned formula (I) are more specifically the following compounds, but the present invention is not limited thereto:
3-[4-(4-chlorophenyl)piperazin-1-yl]methyl-1 H-pyrrolo[2,3-b]pyridine;
3-[4-(4-fluorophenyl)piperazin-1-yl]methyl-1H-pyrrolo[2,3-b]pyridine;
3-[4-(4-bromophenyl)piperazin-1-yl]methyl-1H-pyrrolo[2,3-b]pyridine;
3-[4-(4-iodophenyl)piperazin-1-yl]methyl-1H-pyrrolo[2,3-b]pyridine;
3-[4-(4-trifluoromethylphenyl)piperazin-1-yl]methyl-1H-pyrrolo[2,3-b]pyridine;
3-[4-(4-methylphenyl)piperazin-1-yl]methyl-1H-pyrrolo[2,3-b]pyridine;
3-[4-(4-methoxyphenyl)piperazin-1-yl]methyl-1H-pyrrolo[2,3-b]pyridine;
3-[4-(4-chloro-3-fluorophenyl)piperazin-1-yl]methyl-1H-pyrrolo[2,3-b]pyridine;
3-[4-(2-fluorophenyl)piperazin-1-yl]methyl-1H-pyrrolo[2,3-b]pyridine;
3-[4-(2-methoxyphenyl)piperazin-1-yl]methyl-1H-pyrrolo[2,3-b]pyridine;
3-[4-(3-methoxyphenyl)piperazin-1-yl]methyl-1H-pyrrolo[2,3-b]pyridine;
3-[4-(4-chloro-2-fluorophenyl)piperazin-1-yl]methyl-1H-pyrrolo[2,3-b]pyridine;
3-[4-(4-chloro-2-methoxyphenyl)piperazin-1-yl]methyl-1H-pyrrolo[2,3-b]pyridine;
3-[4-(4-chloro-2-methylphenyl)piperazin-1-yl]methyl-1H-pyrrolo[2,3-b]pyridine;
3-(4-phenylpiperazin-1-yl)methyl-1H-pyrrolo[2,3-b]pyridine;
3-[4-(5-trifluoromethyl-2-pyridyl)piperazin-1-yl]methyl-1H-pyrrolo[2,3-b]pyridine;
3-[4-(3-chloro-5-trifluoromethyl-2-pyridyl)piperazin-1-yl]methyl-1H-pyrrolo[2,3-b]pyridine;
3-[4-(6-chloro-4-trifluoromethyl-2-pyridyl)piperazin-1-yl]methyl-1H-pyrrolo[2,3-b]pyridine;
3-[4-(4-chlorophenyl)piperazine-1-yl]methyl-2-methyl-1H-pyrrolo[2,3-b]pyridine;
3-[4-(4-chlorophenyl)piperazin-1-yl]methyl-1-methyl-1H-pyrrolo[2,3-b]pyridine;
3-[4-(4-ethylphenyl)piperazin-1-yl]methyl-1H-pyrrolo[2,3-b]pyridine;
3-[4-(4-ethoxyphenyl)piperazin-1-yl]methyl-1H-pyrrolo[2,3-b]pyridine;
3-[4-(4-methanesulfonylphenyl)piperazin-1-yl]methyl-1H-pyrrolo[2,3-b]pyridine;
3-[4-(5-chloro-2-pyridyl)piperazin-1-yl]methyl-1H-pyrrolo[2,3-b]pyridine;
3-[4-(5-chloro-2-thienyl)piperazin-1-yl]methyl-1H-pyrrolo[2,3-b]pyridine;
3-[4-(3-isoquinolyl)piperazin-1-yl]methyl-1H-pyrrolo[2,3-b]pyridine;
3-[4-(4-dimethylaminophenyl)piperazin-1-yl]methyl-1H-pyrrolo[2,3-b]pyridine;
3-[4-(phenoxyphenyl)piperszin-1-yl]methyl-1H-pyrrolo[2,3-b]pyridine;
3-[4-(cyclohexyl)piperazin-1-yl]methyl-1H-pyrrolo[2,3-b]pyridine;
3-[4-(benzylphenyl)piperezin-1-yl]methyl-1H-pyrrolo[2,3-b]pyridine;
3-[4-(biphenyl)piperazin-1-yl]methyl-1H-pyrrolo[2,3-b]pyridine;
3-[4-(benzyloxyphenyl)piperazin-1-yl]methyl-1H-pyrrolo[2,3-b]pyridine;
3-[4-(4,4-dimethylcyclohexyl)piperazin-1-yl]methyl-1H-pyrrolo[2,3-b]pyridine;
3-[4-(4-methylthiophenyl)piperazin-1-yl]methyl-1H-pyrrolo[2,3-b]pyridine;
3-[4-(2-methylthiophenyl)piperazin-1-yl]methyl-1H-pyrrolo[2,3-b]pyridine;
3-[4-(4-chlorophenyl)piperazin-1-yl]methylindole;
3-[4-(4-bromophenyl)piperazin-1-yl]methylindole;
3-[4-(4-iodophenyl)piperazin-1-yl]methylindole;
3-[4-(4-chloro-2-fluorophenyl)piperazin-1-yl]methylindole;
3-[4-(4-chlorophenyl)piperazin-1-yl]methyl-4-methylindole;
3-[4-(4-chlorophenyl)piperazin-1-yl]methyl-5-fluoroindole;
3-[4-(4-chlorophenyl)piperazin-1-yl]methyl-6-fluoroindole;
3-[4-(4-chlorophenyl)piperazin-1-yl]methyl-6-methoxyindole;
3-[4-(4-chlorophenyl)piperazin-1-yl]methyl-5-methoxyindole;
3-[4-(4-chlorophenyl)piperazin-1-yl]methyl-4-fluoroindole;
3-[4-(4-chlorophenyl)piperazin-1-yl]methyl-5-cyanoindole;
3-[4-(4-chlorophenyl)piperazin-1-yl]methyl-4-methoxyindole;
5-chloro-3-[4-(4-chlorophenyl)piperazin-1-yl]methylindole;
3-[4-(4-chlorophenyl)piperazin-1-yl]methyl-5-methylindole;
3-[4-(4-chlorophenyl)piperazin-1-yl]methyl-5,6-dimethoxyindole;
3-[4-(5-trifluoromethyl-2-pyridyl)piperazin-1-yl]methylindole;
3-[4-(3-chloro-5-trifluoromethyl-2-pyridyl)piperazin-1-yl]methylindole;
3-[4-(6-chloro-4-trifluoromethyl-2-pyridyl)piperazin-1-yl]methylindole;
3-[4-(5-chloro-2-thienyl)piperazin-1-yl]methylindole;
3-[4-(4-chlorophenyl)piperazin-1-yl]methyl-2-methylindole;
3-[4-(4-chlorophenyl)piperazin-1-yl]methyl-1-methylindole;
3-[4-(4-methylthiophenyl)piperazin-1-yl]methylindole; and
3-[4-(2-methylthiophenyl)piperazin-1-yl]methylindole.

The following compounds may also be used:
3-[4-(4-chloro-3-methoxyphenyl)piperazin-1-yl]methyl-1H-pyrrolo[2,3-b]pyridine;
*N*-{2-chloro-5-[4-(1H-pyrrolo[2,3-b]pyridine-3-ylmethyl)piperazin-1-yl]phenyl}methanesulfonamide;
3-[4-(4-chloro-3-dimethylaminophenyl)piperazin-1-yl]methyl-1H-pyrrolo[2,3-b]pyridine;
3-[4-(4-chloro-2,5-dimethoxyphenyl)piperazin-1-yl]methyl-1H-pyrrolo[2,3-b]pyridine;
*N*-{4-[4-(1H-pyrrolo[2,3-b]pyridine-3-ylmethyl)piperazin-1-yl]phenyl}methanesulfonamide;
*N*-{4-[4-(1H-pyrrolo[2,3-b]pyridine-3-ylmethyl)piperazin-1-yl]phenyl}ethanesulfonamide;
*N*-{3-[4-(1H-pyrrolo[2,3-b]pyridine-3-ylmethyl)piperazin-1-yl]phenyl}methanesulfonamide;
*N*-{3-[4-(1H-pyrrolo[2,3-b]pyridine-3-ylmethyl)piperazin-1-yl]phenyl}ethanesulfonamide;
3-[4-(4-diethylaminophenyl)piperazin-1-yl]methyl-1H-pyrrolo[2,3-b]pyridine;
3-[4-(3-dimethylaminophenyl)piperazin-1-yl]methyl-1H-pyrrolo[2,3-b]pyridine;
3-[4-(4-chloro-3-methoxyphenyl)piperazin-1-yl]methylindole;
*N*-{2-chloro-5-[4-(indole-3-ylmethyl)piperazin-1-yl]phenyl}methanesulfonamide;
3-[4-(4-chloro-3-dimethylaminophenyl)piperazin-1-yl]methylindole;
3-[4-(4-chloro-2,5-dimethoxyphenyl)piperazin-1-yl]methylindole;
*N*-{4-[4-(indol-3-ylmethyl)piperazin-1-yl]phenyl}methanesulfonamide;
*N*-{4-[4-(indol-3-ylmethyl)piperazin-1-yl]phenyl}ethanesulfonamide;
*N*-{3-[4-(indol-3-ylmethyl)piperazin-1-yl]phenyl}methanesulfonamide;
*N*-{3-[4-(indol-3-ylmethyl)piperazin-1-yl]phenyl}ethanesulfonamide;
3-[4-(4-diethylaminophenyl)piperazin-1-yl]methylindole;
3-[4-(3-dimethylaminophenyl)piperazin-1-yl]methylindole;
5-dimethylamino-3-[4-(4-chlorophenyl)piperazin-1-yl]methylindole;
5-methanesulfonyl-3-[4-(4-chlorophenyl)piperazin-1-yl]methylindole;
*N*-{3-[4-(4-chlorophenyl)piperazin-1-ylmethyl]indole-5-yl}methanesulfonamide;
6-methanesulfonyl-3-[4-(4-chlorophenyl)piperazin-1-yl]methylindole;
N-{3-[4-(4-chlorophenyl)piperazin-1-ylmethyl]indole-6-yl}methanesulfonamide;
6-dimethylamino-3-[4-(4-chlorophenyl)piperazin-1-yl]methylindole;
6-hydroxy-3-[4-(4-chlorophenyl)piperazin-1-yl]methylindole; and
*N*-methyl-3-[4-(4-chlorophenyl)piperazin-1-ylmethyl]-5-indolesulfonamide.

The scope of the present invention also includes the use of prodrugs of the aforementioned compounds of formula (I). Ordinarily, such prodrugs are derivatives of compounds of formula (I) which may easily convert to formula (I) compounds *in vivo*. Whether such conversions take place easily *in vivo* can be determined by ordinary enzymatic assays or animal experiments.

When used as pharmaceuticals, the salts of the aforementioned formula (I) compounds are preferably pharmaceutically acceptable salts. Suitable pharmaceutically acceptable salts of the compounds of the present invention include acid addition salts that may be produced, for example, by mixing solutions of compounds of the present invention with solutions of pharmaceutically acceptable acids (for example, hydrochloric acid, sulfuric acid, fumaric acid, maleic acid, succinic acid, acetic acid, benzoic acid, oxalic acid, citric acid, tartaric acid, carbonic acid, or phosphoric acid). Furthermore, when compounds of the present invention have acidic parts, suitable pharmaceutically acceptable salts thereof may include alkali metal salts (for example, sodium salts or potassium salts), alkaline earth metal salts (for example, calcium salts or magnesium salts), and salts formed with suitable organic ligands (for example, quaternary ammonium salts). However, since pharmaceutically unacceptable salts may also be useful in producing the compounds used in the present invention, or pharmaceutically acceptable salts thereof, such salts are also included in the scope of the present invention.

When the compounds used in the present invention have at least one asymmetric center, they may exist as enantiomers. When the compounds used in the present invention have two asymmetric centers, they may exist as diastereomers. All of these isomers and mixtures thereof are included in the scope of the present invention.

Formula (I) compounds can be produced, for example, by methods described in Patent Document 1 (WO94/20497) mentioned above, or methods based on these methods. More specifically, formula (I) compounds may be produced by methods comprising the steps of:
reacting a compound represented by formula (II) 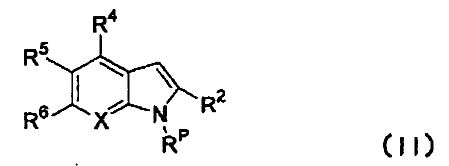 (wherein, X; R², R⁴, R⁵, and R⁶ are defined as above, and R^{p} corresponds to the aforementioned R¹ or is a suitable protecting group),
   with a compound represented by formula (III) 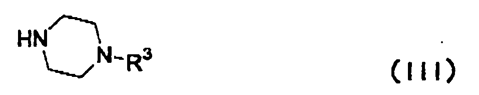 (wherein, R³ is defined as above),
   in the presence of a substantially equimolar amount of formaldehyde;
   next, removing the protecting group R^{p} if necessary;
   and then performing an N-alkylation by standard methods to introduce R¹ if necessary.

This reaction is carried out in an aqueous acetic acid solution, ideally in the presence of a buffer (for example, sodium acetate trihydrate), by stirring the reaction mixture, preferably at room temperature. The formaldehyde may be used in the form of paraformaldehyde or as a formaldehyde solution in an inactive solvent (for example, a 37% aqueous formaldehyde solution).

The protecting group R^{p} is not particularly limited, but is preferably an acyl group (for example, acetyl), and it may be removed by treatment under strongly basic conditions (for example, sodium methoxide in methanol). Alternatively, the protecting group R^{p} may be a carbamoyl group or a tert-butoxycarbonyl group (BOC), and in such cases, the protecting group can be removed by treatment under mildly acidic conditions.

Furthermore, the aforementioned formula (I) compounds may be produced by methods that comprise:
reacting a compound represented by formula (IV) 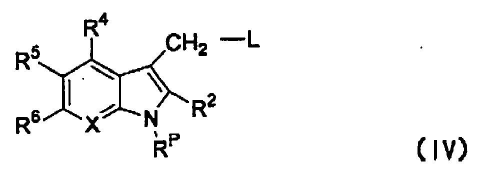 (wherein, X, R², R⁴, R⁵, R⁶, and R^{p} are defined as above, and L is a suitable leaving group),
   with the aforementioned compound of formula (III);
   then removing protecting group R^{p} if necessary;
   and subsequently performing *N*-alkylation using standard methods to introduce R¹ if necessary.

The leaving group L is preferably a halogen atom (for example, a chlorine or bromine) or, a dialkylamino (for example, a dimethylamino). When L is a halogen atom, the reaction between a formula (IV) compound and a formula (III) compound is performed by stirring the reaction mixture in a suitable solvent under basic conditions (for example, potassium carbonate in *N*,*N*-dimethylformamide; or triethylamine in tetrahydrofuran or acetonitrile). When the leaving group L is a dialkylamino, reactions can be performed in an inert solvent (for example, toluene), usually by heating the reaction mixture at the refluxing temperature of the solvent.

The therapeutic or preventive agents of the present invention are effective for ischemic heart disease or ischemic cardiomyopathy including coronary artery disease, angina pectoris, and myocardial infarction. Examples of coronary artery disease include atheromatous coronary artery disease and nonatheromatous coronary artery disease, and more specifically, examples of angina pectoris include effort angina, exertional angina, rest angina, silent myocardial ischemia, and silent angina; myocardial infarction includes acute myocardial infarction. Furthermore, the therapeutic or preventive agents of the present invention are particularly effective for treating or preventing reperfusion injuries such as hemorrhagic infarction or no-reflow phenomenon, which develop immediately after the ischemia-reperfusion that accompanies such diseases.

The therapeutic or preventive agents of the present invention may contain carriers, excipients, or other additives ordinarily used for formulation. By this use, the therapeutic or preventive agents of the present invention can be given as orally administered agents such as tablets, granules, powders, suspensions, capsules, or syrups, or as parenterally administered agents such as injections, suppositories, or isotonic solutions for infusion. The form of administration is preferably intravenous administration, depending on patient condition, and the agents can be solubilized in water by adjusting to a suitable pH. Doses are appropriately adjusted depending on the administration route, as well as patient weight, age, and symptoms. For example, for patients who have developed myocardial infarction, a preferable administration route is continuous intravenous infusion immediately after emergency hospitalization, and for several hours before, during, and after recanalization of the obstructed coronary artery, combined with repeated single administrations depending on urgency. A single administration for an adult is preferably carried out at a dose of 0.01 mg to 500 mg per administration, and continuous administration is preferably carried out at a dose of 0.01 mg to 100 mg per minute.

Solid compositions such as tablets, capsules, powders, granules, and troches may contain binders such as microcrystalline cellulose, gum arabic, gum tragacanth, gelatin, and polyvinylpyrrolidone; excipients such as starch, lactose, and carboxymethyl cellulose; disintegrators such as alginic acid, com starch, and carboxymethylcellulose; lubricants such as magnesium stearate, light anhydrous silicic acid, and colloidal silicon dioxide; sweeteners such as sucrose; flavors such as peppermint and methyl salicylate; and such. Liquid compositions such as syrups or suspensions may contain sorbitol, gelatin, methylcellulose, carboxymethylcellulose, vegetable oils such as peanut oil, and emulsifiers such as lecithin, and if necessary, sweeteners, preservatives, coloring agents, and flavoring agents; and these compositions may be provided as dry formulations. These formulations preferably contain the active ingredient compound at 1 to 95 percent by weight.

For injections, for example, the agents of the present invention may be dissolved in ordinary water for injection in the form of salts, or made into the injectable form of suspensions or emulsions (in medically acceptable oils or mixtures of liquids). In such cases, antibacterial agents such as benzyl alcohol, antioxidants such as ascorbic acid, and medically acceptable buffers or reagents for osmoregulation may also be contained. Such injections preferably contain the active ingredient compound at 0.1 to 8 percent by weight.

All prior art references cited herein are incorporated herein by reference.

### Examples

Hereinafter, Examples of the present invention are described, but the present invention is not to be construed as being limited thereto. First, specific examples of formulations of the therapeutic or preventive agents of the present invention will be presented, but the formulations of the present invention are not limited thereto.

### [Formulation Example 1] (tablets)

(1) 20 mg of active ingredient, (2) 150 mg of lactose, (3) 30 mg of starch, and (4) 6 mg of magnesium stearate. The above ingredients (1) to (4) are shaped into a single tablet.

### [Formulation Example 2] (powders, fine granules, granules)

(1) 20 mg of active ingredient, (2) 180 mg of sugar ester (Daiichi Kogyo Seiyaku Co., product name: DK ester F-160), (3) 15 mg of surfactant (Nikko Chemicals Co., product name: Decaglyn 1-L) and (4) 25 mg of light anhydrous silicic acid. The aforementioned (1) to (4) are mixed to produce powders, which are then granulated into fine granules or granules. These powders, fine granules and granules may also be enclosed in capsules to form capsule formulations.

### [Formulation Example 3] (hard gelatin capsules)

(1) 25 mg of active ingredient, (2) 200 mg of starch, and (3) 10 mg of magnesium stearate. The above ingredients (1) to (3) are formulated into a single hard gelatin capsule by placing them into a hard gelatin capsule.

### [Formulation Example 4] (injections)

(1) 1 mg of active ingredient, (2) 10 mg of glucose, and (3) 2.16 mg of tris(hydroxymethyl)aminomethane. Tris buffer containing the above ingredients (1) to (3) is freeze-dried to prepare injections.

### [Test Example 1] (Examination of drug efficacy on rat models of cardiac ischemia-reperfusion)

3-[4-(4-Chlorophenyl)piperazin-1-yl]methyl-1H-pyrrolo[2,3-b]pyridine was administered to models produced by performing ischemia-reperfusion treatments on rat cardiac coronary arteries, and its effect in reducing cardiac lesions was evaluated histopathologically. The following four groups were formed: a sham-operated group (the steps up until open-chest surgery were performed), an untreated group (open-chest surgery and ischemia-reperfusion were performed, but treatment was not carried out), a 40 mg group and 80 mg group (open-chest surgery and ischemia-reperfusion were performed, and 40 or 80 mg/kg of compound was administered). Five rats were allocated to each group.

The rats (9 to 10-week old male Crj IGS, Japan Charles River Co.) were placed under halothane-nitrous oxide anesthesia and equipped with a ventilator, and by thoracotomy, the left descending coronary artery was exposed to eight cycles of one-minute ischemia and three-minute reperfusion per cycle using polypropylene ligature (PROLENE M-8806, ETHICON); after closing the chest, 60 minutes of subsequent reperfusion was performed. The test compound was administered by dissolving approximately 1 g of the compound in distilled water by adding 7 to 8 mL of 1N HCl, and then orally administering it in a single dose, such that the timing would be four to six hours before model production. Histopathological preparations for evaluation were produced by removing the heart under ether anesthesia after completion of subsequent reperfusion, fixing by soaking in phosphate buffered aqueous neutral formalin solution, and preparing hematoxylin-eosin (HE) double-stained or Tunel-stained preparations according to standard methods.

In evaluation by histopathological examinations, the extent of lesions in the HE preparations were compared, and the frequency of occurrence of Tunel-positive cells (apoptotic cells) were observed in the Tunel preparations. In the observations, the extent of each lesion and frequency of apoptosis occurrence were classified into five levels: "not observed", "slight", "mild", "moderate", or "high". Each of the five classification levels was assigned a score of 0, 0.5, 1.0, 2.0, or 3.0, respectively; and the group averages were calculated for each of the lesions and compared. The histopathological change scores for each of the lesions found in the left ventricular wall of each group are indicated. The results are shown in Table 1.

**Table 1**

| | Sham-operated group | | Untreated group (vehicle) | | 40 mg group | | 80 mg group | |
|---|---|---|---|---|---|---|---|---|
| | Average | SD | Average | SD | Average | SD | Average | SD |
| Edema | 0.0 | 0.0 | 1.4 | 0.5 | 0.6 | 0.5 | 0.1 | 0.2 |
| Hyperemia/congestion | 0.1 | 0.2 | 2.0 | 0.0 | 0.9 | 0.2 | 0.5 | 0.0 |
| Partial bleeding | 0.0 | 0.0 | 1.0 | 0.0 | 0.9 | 0.2 | 0.6 | 0.2 |
| Neutrophil infiltration | 0.0 | 0.0 | 0.3 | 0.3 | 0.0 | 0.0 | 0.0 | 0.0 |
| Edematous degeneration | 0.0 | 0.0 | 2.0 | 0.0 | 1.1 | 0.5 | 0.6 | 0.2 |
| Wavy fiber pattern | 0.0 | 0.0 | 1.4 | 0.5 | 0.6 | 0.3 | 0.1 | 0.2 |
| Contraction band necrosis | 0.2 | 0.3 | 1.4 | 0.5 | 0.6 | 0.2 | 0.6 | 0.2 |
| Coagulation necrosis | 0.0 | 0.0 | 1.6 | 0.5 | 0.7 | 0.3 | 0.5 | 0.4 |
| Necrobiosis | 0.0 | 0.0 | 2.0 | 0.0 | 0.9 | 0.2 | 0.8 | 0.7 |
| Apoptosis | 0.3 | 0.3 | 1.6 | 0.5 | 0.6 | 0.2 | 0.4 | 0.2 |

As described above, the compounds of the present invention showed the effect of suppressing lesions of ischemic heart disease or ischemic cardiomyopathy in rat models of cardiac ischemia reperfusion. These compounds were shown to be effective for suppressing cardiac tissue edema, hyperemia/congestion, bleeding, and neutrophil infiltration. They also reduced the degree or frequency with which myocardial degenerative changes occur, such as the edematous degeneration, wavy fiber pattern, and contraction band necrosis that take place in cardiac tissues. In addition, they reduced the degree or frequency with which coagulation necrosis and necrobiosis occurred. They also reduced the degree or frequency with which apoptosis took place in cardiac tissues.

### Industrial Applicability

The compounds of the present invention are useful as therapeutic or preventive agents for ischemic heart disease or ischemic cardiomyopathy.

## Claims

1. A therapeutic or preventive agent for ischemic heart disease or ischemic cardiomyopathy, which comprises as an active ingredient an upregulator compound for a NAIP gene.

2. The therapeutic or preventive agent of claim 1, wherein the upregulator compound for the NAIP gene is a compound represented by formula (I), or a pharmaceutically acceptable salt thereof: 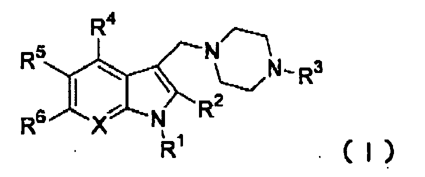 wherein:
X represents CH or a nitrogen;
R¹ and R² independently represent a hydrogen or a C₁₋₆ alkyl;
R³ represents an optionally substituted phenyl, optionally substituted pyridyl, optionally substituted quinolyl, optionally substituted isoquinolyl, optionally substituted pyridazinyl, optionally substituted pyrimidinyl, optionally substituted pyrazinyl, optionally substituted pyranyl, optionally substituted furyl, optionally substituted benzofuryl, optionally substituted dibenzofuryl, optionally substituted thienyl, optionally substituted benzothienyl, optionally substituted indolyl, optionally substituted indazolyl, optionally substituted imidazolyl, optionally substituted benzimidazolyl, optionally substituted oxadiazolyl, optionally substituted thiadiazolyl, or optionally substituted C₃₋₇ cycloalkyl;
R⁴, R⁵, and R⁶ each independently represent a hydrogen, C₁₋₆ alkyl, halogen, cyano, trifluoromethyl, nitro, -OR⁷, -SR⁷, -SO₂R⁷, -SOR⁷, -SO₂NR⁷R⁸, -NR⁷R⁸, -NR⁷SO₂R⁸, -COR⁷, -CO₂R⁷, or -CONR⁷R⁸; and
R⁷ and R⁸ each independently represent a hydrogen or a C₁₋₆ alkyl.

3. The therapeutic or preventive agent of claim 2, wherein
R³ is a phenyl optionally substituted with Y, a pyridyl optionally substituted with Y, a quinolyl optionally substituted with Y, a isoquinolyl optionally substituted with Y, a pyridazinyl optionally substituted with Y, a pyrimidinyl optionally substituted with Y, a pyrazinyl optionally substituted with Y, a pyranyl optionally substituted with Y, a furyl optionally substituted with Y, a benzofuryl optionally substituted with Y, a dibenzofuryl optionally substituted with Y, a thienyl optionally substituted with Y, a benzothienyl optionally substituted with Y, an indolyl optionally substituted with Y, an indazolyl optionally substituted with Y, an imidazolyl optionally substituted with Y, a benzimidazolyl optionally substituted with Y, an oxadiazolyl optionally substituted with Y, a thiadiazolyl optionally substituted with Y, or a C₃₋₇ cycloalkyl optionally substituted with Y;
Y is at least one substituent selected from the group consisting of a C₁₋₆ alkyl, phenyl, phenoxy, benzyl, benzyloxy, halogen, trifluoromethyl, nitro, cyano, -OR⁹, -SR⁹, -SO₂R⁹, -SOR⁹, -SO₂NR⁹R¹⁰, -NR⁹R¹⁰, -NR⁹SO₂R¹⁰, -COR⁹, -CO₂R⁹, and -CONR⁹R¹⁰; and
R⁹ and R¹⁰ each independently represent a hydrogen or a C₁₋₆ alkyl.

4. The therapeutic or preventive agent of claim 2 or 3, wherein R³ is a phenyl optionally substituted with Y, a pyridyl optionally substituted with Y, an isoquinolyl optionally substituted with Y, a thienyl optionally substituted with Y, or a C₃₋₇ cycloalkyl optionally substituted with Y

5. The therapeutic or preventive agent of any one of claims 2 to 4, wherein
R³ is a phenyl optionally substituted with Y

6. The therapeutic or preventive agent of any one of claims 2 to 5, wherein X represents a nitrogen.

7. The therapeutic or preventive agent of any one of claims 1 to 6, which comprises a 3-[4-(4-chlorophenyl)piperazin-1-yl]methyl-1H-pyrrolo[2,3-b]pyridine or a salt thereof as an active ingredient.

8. A method for treating or preventing ischemic heart disease or ischemic cardiomyopathy, which comprises administering an effective dose of an upregulator compound for a NAIP gene to a patient

9. Use of an upregulator compound for a NAIP gene for producing a therapeutic or preventive agent for ischemic heart disease or ischemic cardiomyopathy.

10. A therapeutic or preventive agent for ischemic heart disease or ischemic cardiomyopathy, which comprises as an active ingredient, any of the following substances:
(a) a NAIP protein;
(b) a NAIP protein mutant functionally equivalent to a NAIP protein and comprising an amino acid sequence with one or more amino acid deletions, substitutions, or additions in the amino acid sequence of a NAIP protein; and
(c) a DNA encoding the protein of (a) or (b), or a vector expressing the protein.

11. A method for treating or preventing ischemic heart disease or ischemic cardiomyopathy which comprises administering to a patient an effective dose of any of the following substances:
(a) a NAIP protein;
(b) a NAIP protein mutant functionally equivalent to a NAIP protein and comprising an amino acid sequence with one or more amino acid deletions, substitutions, or additions in the amino acid sequence of a NAIP protein; and
(c) a DNA encoding the protein of (a) or (b), or a vector expressing the protein.

12. Use of any of the substances of (a) to (c) below for producing a therapeutic or preventive agent for ischemic heart disease or ischemic cardiomyopathy:
(a) a NAIP protein;
(b) a NAIP protein mutant functionally equivalent to a NAIP protein and comprising an amino acid sequence with one or more amino acid deletions, substitutions, or additions in the amino acid sequence of a NAIP protein; and
(c) a DNA encoding the protein of (a) or (b), or a vector expressing the protein.
